# EUROPÄISCHE PATENTSCHRIFT

(11) **EP 2 326 272 B1**
(45) Veröffentlichungstag und Bekanntmachung des Hinweises auf die Patenterteilung: **18.04.2012**
(21) Anmeldenummer: 09749529.5
(22) Anmeldetag: 23.04.2009
(51) Int. Cl.: A61B 18/02, A61B 10/02

(54) **KRYOCHIRURGISCHES INSTRUMENT ZUR GEWINNUNG EINER GEWEBEPROBE**
CRYOSURGICAL INSTRUMENT FOR OBTAINING A TISSUE SAMPLE
INSTRUMENT CRYOCHIRURGICAL POUR L'OBTENTION D'UN ÉCHANTILLON DE TISSU

(30) Priorität: 23.05.2008 DE 102008024946
(43) Veröffentlichungstag der Anmeldung: 01.06.2011
(73) Patentinhaber: ERBE Elektromedizin GmbH, 72072 Tübingen (DE)
(72) Erfinder: FISCHER, Klaus, 72202 Nagold (DE); SCHÄLLER, Daniel, 72070 Tübingen (DE); VOIGTLÄNDER, Matthias, 72810 Gomaringen (DE); SZYRACH, Mara, 72070 Tübingen (DE); SIGLE, Irina, 72116 Mössingen (DE); BLOBEL, Lars, 72119 Ammerbuch-Entringen (DE); ENDERLE, Markus, 72070 Tübingen (DE)
(74) Vertreter: Bohnenberger, Johannes
(86) Internationale Anmeldenummer: PCT/EP2009/002971
(87) Internationale Veröffentlichungsnummer: WO 2009/141039

(56) Entgegenhaltungen:
- WO-A-00/32126
- WO-A-98/46148
- US-A- 4 211 231
- US-A1- 2002 143 323
- US-A1- 2005 288 657
- US-B1- 6 551 309

## Beschreibung

Die Erfindung betrifft ein kryochirurgisches Instrument zur Gewinnung einer Gewebeprobe gemäß dem Oberbegriff des Patentanspruchs 1, der aus dem Dokument US-B1 6551309 bekannt ist.

In der Kryochirurgie wird unter anderem durch den gezielten Einsatz von Kälte eine Gewebeprobenentnahme (Biopsie) ermöglicht. Dabei wird eine Kryosonde über ein Endoskop in die Nähe eines bestimmten Gewebebereichs gebracht. Während der Sondenkopf das zu entnehmende Gewebe kontaktiert, wird dieser derart abgekühlt, dass das Gewebe zumindest abschnittsweise an dem Sondenkopf festfriert. Nach einem Abtrennen des umliegenden Gewebes kann die Probe (Biopsat) in das Endoskop eingezogen und Untersuchungen zugänglich gemacht werden.

Die Kühlleistung kann beispielsweise durch das gezielte Einsetzen des Joule-Thomson-Effekts bereitgestellt werden. Hierbei erfährt ein Fluid, insbesondere ein Gas durch Drosselung (Druckänderung), eine Temperaturänderung. In der Kryochirurgie wird Gas unter hohem Druck in eine Expansionskammer derart expandiert, dass sich dessen Volumen vergrößert. Dabei erhöht sich der mittlere Teilchenabstand des Fluids, wodurch die dessen Temperatur sinkt.

Eine entsprechende Kryosonde ist aus der US 7,156,840 B2 bekannt. Derartige Kryosonden sollen auch in dünnen Hohlorganen wie z.B. Gallen- oder Pankreasgängen eingesetzt werden, um eine Histologie des Gewebes vorzunehmen.

Ein wichtiges Kriterium für die Biopsie ist die Qualität der entnommenen Probe. Es sollte hierbei zu keiner mechanischen Verformung der Probe kommen.

Um im Gallen- und Pankreasgang unter endoskopischer Sicht zu arbeiten, werden flexible Mikro-Endoskope eingesetzt. Der Außendurchmesser dieser flexiblen Mikro-Endoskope ist sehr gering, beispielsweise ≤ 3 mm. In den Arbeitskanal derartiger Endoskope können lediglich Instrumente mit einem wesentlich geringeren Außendurchmesser eingeführt werden. Häufig darf der Außendurchmesser der hier verwendeten Instrumente einen Millimeter nicht überschreiten.

Des Weiteren sind in diesem Einsatzbereich die Distanzen zwischen Eintrittsöffnung des Endoskops und Arbeitsbereich sehr lang. Entsprechend lang ist auch der Arbeitskanal des Endoskops. Das in das Endoskop eingeführte Instrument, beispielsweise das kryochirurgische Instrument mit Kryosonde muss daher entsprechende Abmessungen aufweisen. Beispielsweise kann eine Länge von 180 cm gefordert sein.

Eine weitere Anforderung für den Einsatz einer Kryosonde in dem oben beschriebenen Spezialgebiet ist das Bereitstellen von ausreichender Kühlleistung, so dass das Biopsat mit ausreichender Sicherheit an der Sondenspitze fixiert werden kann. Die Kühlleistung wird maßgeblich durch die Druckdifferenz in der Expansionskammer bestimmt. Das heißt, je höher die Differenz zwischen dem Druck des Gases vor dem Austritt über eine Düsenöffnung und dem Druck innerhalb der Expansionskammer ist, umso höher ist auch die Kühlleistung. Je geringer die entsprechende Gaszuleitung dimensioniert ist, umso schwieriger ist es, einen ausreichend hohen Druck vor der Düsenöffnung bereitzustellen. Des Weiteren muss das ausströmende Gas aus der Expansionskammer abgeführt werden, um das Druckgefälle aufrecht zu erhalten. Hierfür werden üblicherweise Gasrückführungen mit einem ausreichend großen Durchmesser vorgesehen. Mit geringerem Durchmesser der Rückführungsleitungen steigt jedoch der Strömungswiderstand und somit der Druck innerhalb der Expansionskammer.

Aus diesen Gründen erweist es sich als äußerst schwierig, Kryosonden bereitzustellen, die die geforderten Abmessungen haben und gleichzeitig eine ausreichende Leistung bereitstellen. Häufig greift man daher auf herkömmliche Biopsiemethoden zurück. Diese umfassen beispielsweise die Gewinnung von Proben mittels einer Bürste, oder die Nadelbiopsie.

Jedoch ist die Qualität der dadurch gewonnenen Proben üblicherweise wesentlich geringer als die, die durch eine Kryobiopsie erzielt werden kann.

Ausgehend von diesem Stand der Technik ist es Aufgabe der vorliegenden Erfindung, ein verbessertes kryochirurgisches Instrument bereitzustellen. Insbesondere soll ein kryochirurgisches Instrument zur Gewinnung einer Gewebeprobe bereitgestellt werden, das sich zur Verwendung in dünnen Hohlorganen eignet. Des Weiteren soll ein entsprechendes Verfahren zur Abkühlung eines Sondenkopfs einer kryochirurgischen Sonde angegeben werden.

Diese Aufgabe wird erfindungsgemäß durch eine Vorrichtung nach Anspruch 1 gelöst.

Insbesondere wird die Aufgabe durch ein kryochirurgisches Instrument zur Gewinnung einer Gewebeprobe mit einer Sonde gelöst, wobei die Sonde umfasst:
- einen Sondenkopf zum Anfrieren von Gewebe;
- einen Sondenschaft zum Heranführen des Sondenkopfs an das Gewebe;
- eine Zuführeinrichtung zum Zuführen eines Fluids, insbesondere eines Gases in eine Expansionskammer;
- eine Abführeinrichtung, die an die Expansionskammer zum Abführen des Fluids angeschlossen ist, wobei die Expansionskammer derart ausgebildet ist, dass das zugeführte Fluid zum Abkühlen des Sondenkopfs expandiert.

Die Abführeinrichtung umfasst mindestens eine Öffnung am Distalbereich der Sonde, die das Fluid in einen Außenbereich außerhalb der Sonde abführt.

Ein wesentlicher Punkt der Erfindung besteht darin, dass die Rückführung des Fluids nur teilweise oder überhaupt nicht innerhalb der Sonde erfolgt. Das über die Zuführeinrichtung zugeführte Gas breitet sich in der Expansionskammer aus und wird von dort in einen Außenbereich außerhalb der Sonde abgeführt. Somit kann auf das Vorsehen einer Rückführleitung innerhalb der Sonde teilweise oder gänzlich verzichtet werden. Die Zuführeinrichtung kann entsprechend größer dimensioniert sein, um ausreichend Druck in der Expansionskammer bereitzustellen. Häufig sind die austretenden Fluidmengen derart gering, dass diese innerhalb der Organe keine Schäden verursachen. Auch das verwendete Fluid, stellt kein Risiko für den Patienten dar. Soweit das Fluid dennoch abgeführt werden soll, kann hierfür ein getrennter Mechanismus vorgesehen werden. Der Arbeitskanal des verwendeten Endoskops kann beispielsweise zum Abführen des Fluids verwendet werden.

Bei der erfindungsgemäßen Sonde definiert der Sondenkörper einen Außenbereich und einen Innenbereich. Es ist offensichtlich, dass auch bei herkömmlichen Sonden eine Abführung des Fluids aus dem Innenbereich in den Außenbereich erfolgt. Jedoch befinden sich die entsprechenden Mittel nicht am Distalbereich der Sonde, sondern in deren proximalem Bereich.

Das kryochirurgische Instrument kann mindestens eine Öffnung am distalen Ende des Sondenschafts und/oder im Sondenkopf umfassen. Das heißt, der Distalbereich ist derart definiert, dass hierunter Sondenkopf und das distale Ende des Sondenschafts fällt.

Das kryochirurgische Instrument umfasst einen Adapter zum Ableiten des Fluids in proximaler Richtung. Ein derartiger Adapter kann beispielsweise das aus der Sonde austretende Fluid in den Arbeitskanal eines Endoskops einleiten. Des Weiteren kann der Adapter derart ausgestaltet sein, dass er eine Abführeinrichtung für das Fluid bereitstellt. Diese Abführeinrichtung führ.t, wie herkömmliche kryochirurgische Instrumente, das Fluid zum proximalen Ende der Sonde.

Der Adapter umfasst einen Schutzschlauch mit einer Dichtung, wobei die Sonde in dem Schutzschlauch beweglich gelagert ist und die Dichtung einen Innenraum oder Innenbereich des Schutzschlauches zur Aufnahme des Fluids gegenüber der Sonde abdichtet. Das Fluid wird also über die Zuführeinrichtung in die Expansionskammer eingeleitet, tritt von dort über mindestens eine Öffnung in den Außenbereich der Sonde, wobei die Sonde von dem Schutzschlauch derart umschlossen wird, dass ein Zwischenraum zwischen Sonde und Schutzschlauch entsteht. In diesen Zwischenraum kann das Fluid in proximaler Richtung geführt werden. Die Dichtung am distalen Ende des Schutzschlauches sorgt dafür, dass ein Entweichen des Fluids in distale Richtung nicht möglich ist.

Der Schutzschlauch kann einen Aufnahmebereich für die Gewebeprobe umfassen. Erfindungsgemäß kann die Gewebeprobe, die an dem Sondenkopf befestigt ist, also derart eingezogen werden, dass sie innerhalb des Schutzschlauches zu liegen kommt. Dadurch kann die Gewebeprobe vor äußeren Einflüssen, insbesondere mechanischen Belastungen geschützt werden.

Allgemein kann der Schutzschlauch dazu dienen, die erfindungsgemäße Sonde derart umzurüsten, dass sie auch in Bereichen eingesetzt werden kann, in denen das Austreten von Fluid nahe dem Operationsbereich unerwünscht ist.

Wie bereits beschrieben, kann der Adapter zur Einleitung des Fluids in einen Arbeitskanal eines Endoskops angepasst sein.

Die mindestens eine Öffnung am Distalbereich der Sonde kann seitlich des Sondenschafts angeordnet sein oder in Form eines Partikelfilters den Sondenkopf bilden. Der Partikelfilter kann beispielweise Öffnungen mit dem Durchmesser von ca. 4 µm haben. Ein wesentlicher Gedanke der Erfindung besteht also auch hier darin, dass bei dem Kühlverfahren das Fluid im Distalbereich abgeleitet wird und dadurch keine getrennte Rückführung des Fluids nötig ist. Bei dem Verfahren kann das Zuführen des Fluids über eine Zeitspanne erfolgen, die ≤ 5 Sekunden ist. Dadurch werden nur sehr geringe Gasmengen freigesetzt, die in den Außenbereich der Sonde gelangen.

Weitere vorteilhafte Ausführungsformen ergeben sich anhand der Unteransprüche.

Nachfolgend wird die Erfindung anhand von einigen Ausführungsbeispielen beschrieben, die mittels Abbildungen näher erläutert werden. Hierbei zeigen:
- - Fig. 1: eine schematische Darstellung eines kryochirurgischen Instruments;
- - Fig. 2: eine schematische Darstellung des kryochirurgischen Instruments aus Fig. 1 innerhalb eines Endoskops im klinischen Einsatz;
- - Fig. 3: eine schematische Darstellung des distalen Endes einer starren Kryosonde gemäß dem Stand der Technik;
- - Fig. 4: eine schematische Darstellung des distalen Endes einer flexiblen Kryosonde gemäß dem Stand der Technik;
- - Fig. 5: eine schematische Darstellung des distalen Endes einer Kryosonde;
- - Fig. 6: eine schematische Darstellung des distalen Endes einer flexiblen Kryosonde gemäß dem Stand der Technik;
- - Fig. 7: eine schematische Darstellung des distalen Endes einer Kryosonde;
- - Fig. 8: eine schematische Darstellung des distalen Endes einer ersten Ausführungsform der erfindungsgemäßen Kryosonde mit Schutzschlauch;
- - Fig. 9: eine erfindungsgemäße Kryosonde im Arbeitskanal eines flexiblen Endoskops;
- - Fig. 10: eine erfindungsgemäße Kryosonde mit Schutzschlauch und Biopsat;
- - Fig. 11: die erfindungsgemäße Kryosonde aus Fig. 10 mit eingezogenem Biopsat; und
- - Fig. 12: eine schematische Darstellung des distalen Endes einer Kryosonde.

In der nachfolgenden Beschreibung werden für gleiche und gleich wirkende Teile dieselben Bezugsziffern verwendet.

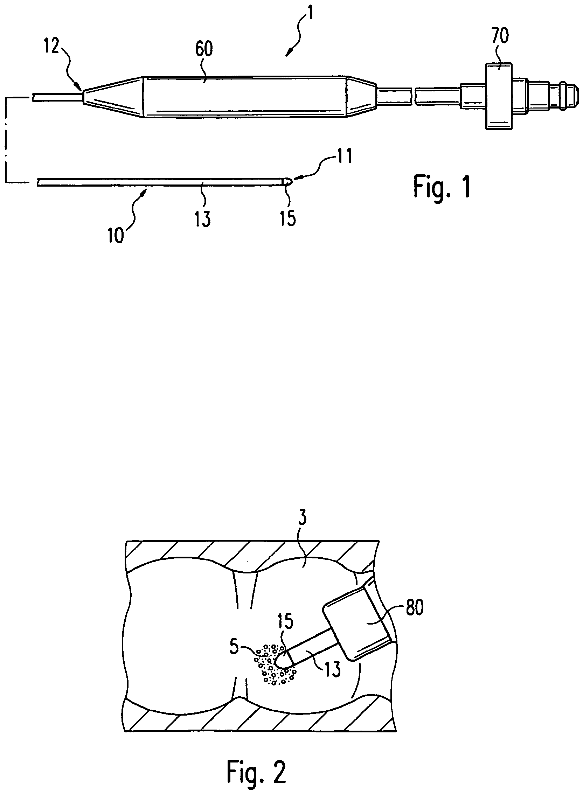

Fig. 1 zeigt schematisch den Aufbau eines kryochirurgischen Instruments 1. Dieses umfasst einen Handgriff 60 zum Führen der Kryosonde 10, wobei sich die Kryosonde 10 aus einem Schaft 13 und einem Sondenkopf 15 zusammensetzt. Der Sondenkopf 15 bildet das distale Ende 11 der Kryosonde 10. Das proximale Ende 12 der Kryosonde 10 setzt direkt am Handgriff 60 an. Am proximalen Ende des Handgriffs 60 befindet sich ein Anschluss mittels dessen sich das kryochirurgische Instrument 1 an eine Druckluftquelle anschließen lässt. Die Druckluftquelle versorgt das kryochirurgische Instrument 1 mit Druckluft.

Fig. 2 zeigt die Kryosonde 10 aus Fig. 1 im klinischen Einsatz. Der Schaft 13 der Kryosonde 10 befindet sich innerhalb des Arbeitskanals eines Endoskops 80, wobei das Endoskop 80 in ein Hohlorgan, beispielsweise einen Abschnitt des Darms, eingeführt ist. Der Schaft 13 sowie der Sondenkopf 15 überragen das distale Ende des Endoskops 80 und kontaktieren einen Bereich des Gewebes 3 des Darms. Der Sondenkopf 15 kann mittels des Joule-Thomson-Effekts derart abgekühlt werden, dass der besagte Bereich an dem Sondenkopf 15 festfriert. Durch einen mechanischen Zug lässt sich eine Gewebeprobe 5 aus dem Gewebe 3 heraustrennen.

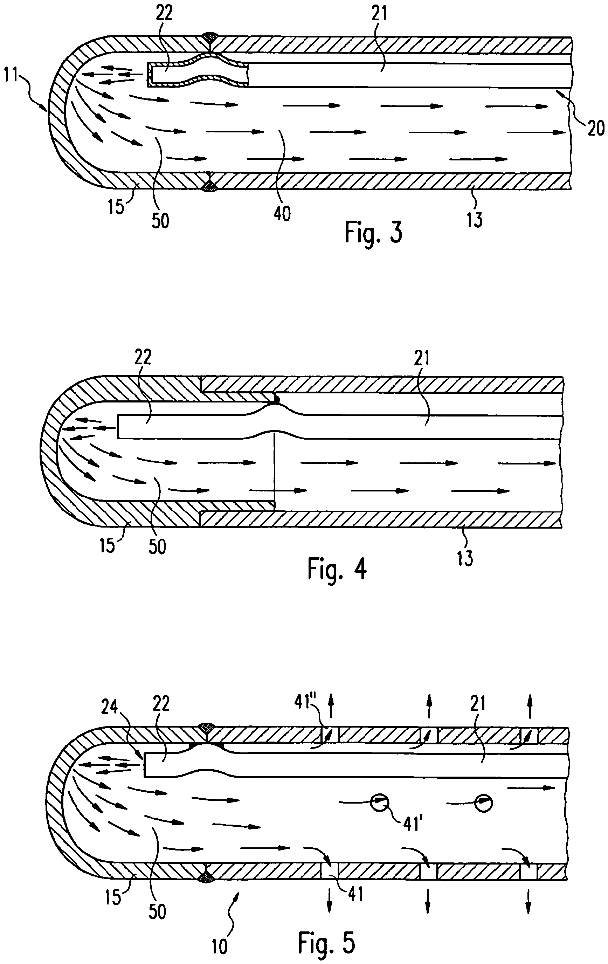

Fig. 3 zeigt das distale Ende einer Kryosonde 10 im Querschnitt. Man unterscheidet üblicherweise zwischen starren Kryosonden 10 (Fig. 3) und flexiblen Kryosonden 10 (Fig. 4). Die Kryosonden gemäß den Fig. 3 und 4 umfassen einen Sondenkopf 15, der das distale Ende 11 der Krysonde 10 bildet. Dieser Sondenkopf 15 ist an dem Sondenschaft 13 befestigt. Sondenkopf und Sondenschaft 13 umschließen einen Bereich, der im Weiteren als Innenbereich der Sonde 10 bezeichnet wird. In diesem Innenbereich befindet sich eine Gaszuführungseinrichtung 20, die eine Gaszuführungsleitung 21 und ein Distalende 22 der Gaszuführungsleitung 21 umfasst. Über diese Gaszuführungsleitung wird Gas unter hohem Druck in eine Expansionskammer im Innenraum des Sondenkopfs 15 gebracht. Das Gas tritt über eine Austrittsöffnung in diese Expansionskammer 50 aus und entzieht aufgrund des Joule-Thomson-Effekts dem Sondenkopf 15 Wärmeenergie. Der unausgefüllte Innenraum der Kryosonde 10 bildet eine Gasrückführungseinrichtung 40. Das heißt, das austretende Gas wird im Innenraum der Sonde 10 in proximale Richtung, also in Richtung des Anschlusses 70 abgeführt.

Fig. 5 zeigt das distale Ende 11 einer Kryosonde 10. Auch diese Kryosonde 10 setzt sich aus einem Schaft 13 und einem Sondenkopf 15 zusammen, die miteinander verklebt sind. Schaft 13 und Sondenkopf 15 bilden einen Außenmantel der Kryosonde 10. Innerhalb der Kryosonde 10 befindet sich die Gaszuführungsleitung 21, die über eine Düsenöffnung 24 am distalen Ende 22 der Gaszuführleitung 21 Gas unter hohem Druck in die Expansionskammer 50 strömen lässt. Die Gasrückführungseinrichtung 40 umfasst Öffnungen 41 bis 41", die durch eine Perforation des Außenmantels der Kryosonde 10 hergestellt sind. Das expandierte Gas kann über diese Öffnungen 41 bis 41" aus dem Innenbereich der Kryosonde 10, insbesondere aus der Expansionskammer 50 in den Außenbereich der Kryosonde 10 entweichen. Die Öffnungen 41 bis 41" befinden sich nahe dem distalen Ende 11 der Kryosonde 10.

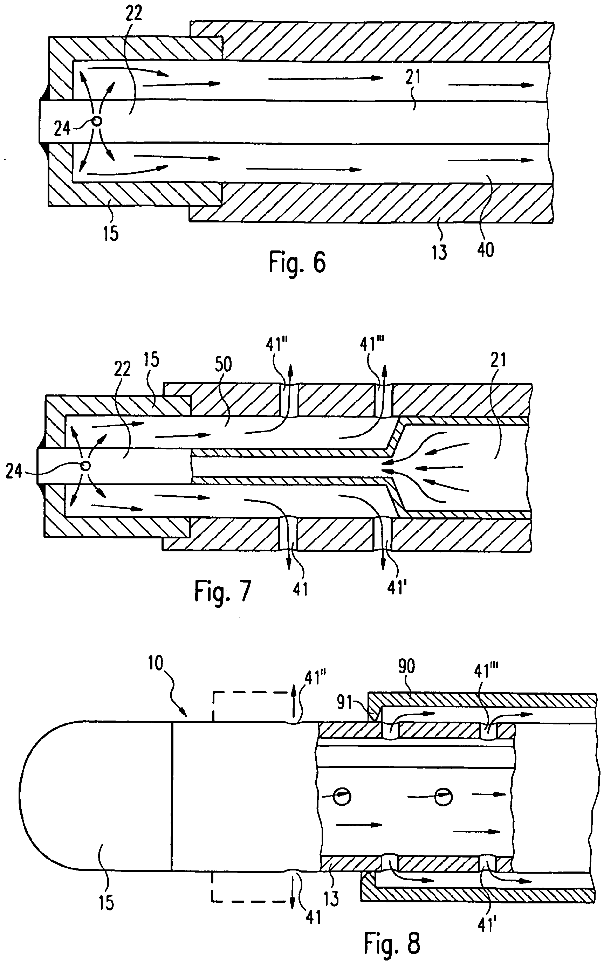

Die Fig. 6 zeigt eine weitere Kryosonde 10. Entlang der Längsachse, im Innenbereich der Kryosonde 10 erstreckt sich die Gaszuführungsleitung 21, die an ihrem distalen Ende 22 verschlossen ist und einen Teil des Sondenkopfs 15 bildet. Nahe dem distalen Ende 22 der Gaszuführungsleitung 21 befindet sich eine seitliche Düsenöffnung 24, über die das Gas in den Innenraum der Kryosonde 10 strömt. Der Zwischenraum zwischen Gaszuführungsleitung 21 und Schaft 13 der Kryosonde 10 bildet die Gasrückführungseinrichtung 40.

Fig. 7 zeigt eine Kryosonde 10. In ihrem allgemeinen Aufbau gleicht die Kryosonde 10 gemäß Fig. 7 dem der Fig. 6. Jedoch nimmt die Gaszuführungsleitung 21 bis auf einen kurzen Abschnitt nahe dem distalen Ende 22 der Gaszuführungsleitung 21 den gesamten Innenbereich des Schafts 13 der Kryosonde 10 ein. Nahe dem besagten distalen Ende 22 verengt sich die Gaszuführungsleitung 21 derart, dass ein Zwischenraum zwischen Gaszuführungsleitung 21 und Schaft 13 bzw. Sondenkopf 15 entsteht. Dieser Zwischenraum dient als Expansionskammer 50, in die über die seitliche Düsenöffnung 24 Gas aus der Gaszuführungsleitung 21 einströmt. Dieser Zwischenraum bildet des Weiteren einen Teil der Gasrückführungseinrichtung 40, die das expandierte Gas über Öffnungen 41 bis 41"' in den Außenbereich der Sonde ableitet. Auf eine Gasrückführungseinrichtung 40 im proximalen Bereich des Schafts 13 kann verzichtet werden.

Fig. 8 zeigt ein erstes Ausführungsbeispiel der erfindungsgemäßen Kryosonde 10. Diese Kryosonde 10 entspricht in ihrem Aufbau im Wesentlichen dem der Kryosonde 10 aus Fig. 5. Die Gasrückführungseinrichtung 40 wird teilweise durch den Innenraum der Kryosonde 10 sowie durch die Öffnungen 41 bis 41"' gebildet. Die Kryosonde 10 befindet sich innerhalb eines Schutzschlauches 90, mittels dessen das Gas gezielt in proximale Richtung abgeleitet werden kann. Am distalen Ende des Schutzschlauches 90 befindet sich eine Ringdichtung 91, die an dem Schaft 13 der Kryosonde 10 anliegt und den Innenbereich des Schutzschlauches 90 gegenüber dem Außenbereich distal abdichtet. Der Schutzschlauch 90 und die Ringdichtung 91 sind derart ausgebildet, dass die Kryosonde 10 innerhalb des Schutzschlauches 90 in distale und proximale Richtung bewegt werden kann. Je nach Position der Kryosonde 10 innerhalb des Schutzschlauches 90 und je nach Position der Öffnungen 41 bis 41"' wird das expandierte Gas vollständig über den Innenbereich des Schutzschlauches 90 abgeleitet oder nur teilweise. In der Position gemäß Fig. 8 entweicht das Gas über die Öffnungen 41, 41" unmittelbar in das Organ, während das Gas, das durch die Öffnungen 41' und 41"' entweicht, in den Innenbereich des Schutzschlauches 90 eindringt und dort in proximale Richtung abgeleitet wird.

Es ist offensichtlich, dass die erfindungsgemäßen Sonden einen wesentlich dünneren Durchmesser aufweisen können, da der Innenraum der Kryosonden 10 vollständig oder zu einem großen Prozentsatz durch die Gaszuführungsleitung 21 ausgefüllt sein kann. Durch die Verwendung des Schutzschlauches 90 ist es möglich, die erfindungsgemäßen Sonden auch bei Anwendungen einzusetzen, in denen das unmittelbare Entweichen des Gases in ein Organ oder Gewebe nicht gewünscht ist.

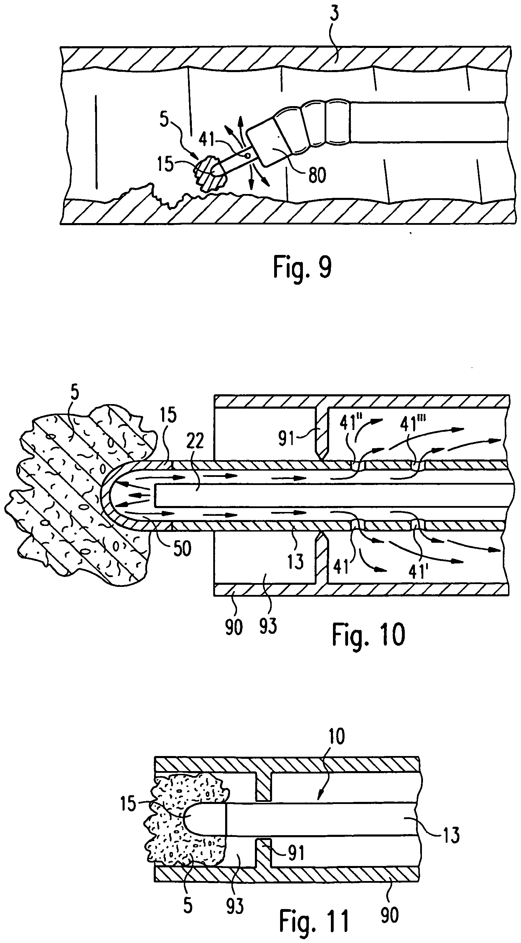

Jedoch gibt es zahlreiche Anwendungsbereiche, in denen das Entweichen von geringen Mengen von Gas unschädlich ist. Beispielsweise zeigt Fig. 9 eine erfindungsgemäße Kryosonde 10 in dem Arbeitskanal eines Endoskops 80, das in einen Darmtrakt eingeführt ist. Das zum Festeisen einer Gewebeprobe 5 nötige Gas entweicht hier über die Öffnung 41 unmittelbar in den Darmtrakt. Hierdurch wird der Patient nicht gefährdet.

Die Fig. 10 und 11 zeigen eine Weiterbildung des Schutzschlauchs 90 aus Fig. 8. Der Schutzschlauch 90 mit der Ringdichtung 91 dient nach wie vor dazu, das über die Öffnungen 41 bis 41"' austretende Gas zumindest teilweise abzuführen. Jedoch umfasst der Schutzschlauch 90 der Figuren 10 und 11 eine Probenkammer 93 am distalen Ende des Schutzschlauches.

Die Probenkammer 93 dient zur Aufnahme einer Gewebeprobe 5. Die Kryosonde 10 lässt sich derart in das Innere des Schutzschlauches 90 zurückziehen, dass die Gewebeprobe 5 ebenfalls innerhalb des Schutzschlauches, nämlich in der Probenkammer 93 zu liegen kommt. Hierdurch wird ein Abstreifen der Probe beim Herausziehen der Kryosonde 10 aus dem Arbeitskanal des Endoskops 80 vermieden.

Die Probenkammer 93 wird in den Ausführungsbeispielen der Fig. 10 und 11 dadurch gebildet, dass sich die Ringdichtung 91 nicht unmittelbar am distalen Ende des Schutzschlauches 90 befindet, sondern in proximale Richtung leicht versetzt ist. Hierdurch ist ein Abschnitt des Innenbereichs des Schutzschlauchs 90 zur Aufnahme der Gewebeprobe 5 in distale Richtung offen.

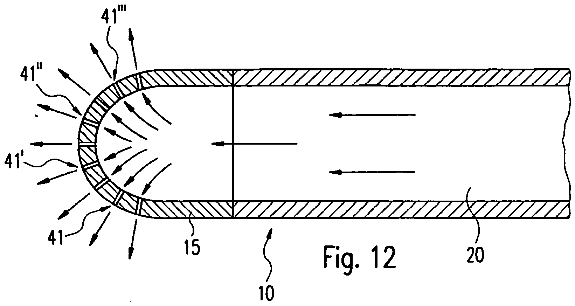

Fig. 12 zeigt eine Kryosonde 10. Die Öffnungen 41 bis 41"' befinden sich unmittelbar am distalen Ende 11 der Kryosonde 10. Sie werden durch einen Partikelfilter mit geringen Lochdurchmesser am Sondenkopf 15 gebildet. Die Öffnungen 41 bis 41" fallen mit dem Bereich zusammen, der die Gewebeprobe 5 aufnimmt. Der gesamte Innenraum der Kryosonde 10 wird als Gaszuführungseinrichtung 20 genutzt. Der Außenraum unmittelbar vor dem Sondenkopf 15 dient in diesem Ausführungsbeispiel als Expansionskammer 50. Die Öffnungen 41 bis 41"' haben somit eine Doppelfunktion. Zum einen sind sie Düse für die Expansion des Gases zum anderen Teil der Gasrückführungseinrichtung 40.

### Bezugszeichenliste

- 1: Kryochirurgisches Instrument
- 3: Gewebe
- 5: Gewebeprobe
- 10: Kryosonde
- 11: distales Ende der Kryosonde
- 12: proximales Ende der Kryosonde
- 13: Schaft
- 15: Sondenkopf
- 20: Gaszuführungseinrichtung
- 21: Gaszuführungsleitung
- 22: distales Ende der Gaszuführungsleitung
- 24: Düsenöffnung
- 40: Gasrückführungseinrichtung
- 41, 41', 41", 41"': Öffnung
- 50: Expansionskammer
- 60: Handgriff
- 70: Anschluss
- 80: Endoskop
- 90: Schutzschlauch
- 91: Ringdichtung
- 93: Probenkammer

## Patentansprüche

1. Kryochirurgisches Instrument zur Gewinnung einer Gewebeprobe (5) mit einer Sonde (10), wobei die Sonde (10) umfasst:
- einen Sondenkopf (15) zum Anfrieren von Gewebe (3);
- einen Sondenschaft (13) zum Heranführen des Sondenkopfs (15) an das Gewebe (3);
- eine Zuführeinrichtung (20) zum Zuführen eines Fluids in eine Expansionskammer (50) des Instruments;
- eine Abführeinrichtung (40), die an die Expansionskammer (50) zum Abführen des Fluids angeschlossen ist, wobei die Expansionskammer (50) derart ausgebildet ist, dass das zugeführte Fluid darin zur Abkühlung des Sondenkopfs (15) expandiert,
wobei die Abführeinrichtung (40) mindestens eine Öffnung (41 - 41"') am Distalbereich der Sonde (10) umfasst, die das Fluid in einen Außenbereich außerhalb der Sonde (10) abführt, und das kryochirurgische Instrument einen Adapter zum Ableiten des Fluids in proximale Richtung umfasst **gekennzeichnet dadurch, dass** der Adapter
einen Schutzschlauch (90) mit einer Dichtung (91) umfasst, wobei die Sonde (10) in dem Schutzschlauch (90) beweglich gelagert ist und die Dichtung (91) einen Innenraum des Schutzschlauches (90) zur Aufnahme des Fluids gegenüber der Sonde (10) distal abdichtet.

2. Kryochirurgisches Instrument nach Anspruch 1,
**dadurch gekennzeichnet, dass**
die Abführeinrichtung (40) mindestens eine Öffnung (41-41"') am distalen Ende des Sondenschafts (13) und/oder im Sondenkopf (15) umfasst.

3. Kryochirurgisches Instrument nach einem der vorhergehenden Ansprüche,
**dadurch gekennzeichnet, dass**
die Expansionskammer (50) zur Abkühlung des Sondenkopfs (15) innerhalb der Sonde (10) angeordnet ist.

4. Kryochirurgisches Instrument nach einem der vorhergehenden Ansprüche,
**dadurch gekennzeichnet, dass**
der Schutzschlauch (90) einen Aufnahmebereich (93) für die Gewebeprobe (5) umfasst.

5. Kryochirurgisches Instrument nach einem der vorhergehenden Ansprüche,
**dadurch gekennzeichnet, dass**
der Adapter zur Einleitung des Fluids in einen Arbeitskanal eines Endoskops (80) angepasst ist.

6. Kryochirurgisches Instrument nach einem der vorhergehenden Ansprüche,
**dadurch gekennzeichnet, dass**
die Zuführeinrichtung (20) eine Zuführleitung (21) umfasst, die im Inneren des Sondenschafts (13) angeordnet ist.

7. Kryochirurgisches Instrument nach einem der vorhergehenden Ansprüche,
**dadurch gekennzeichnet, dass**
die Abführeinrichtung (40) einen Partikelfilter an dem Sondenkopf (15) umfasst.

## Claims

1. Cryosurgical instrument for obtaining a tissue sample (5) using a probe (10), wherein the probe (10) comprises:
- a probe head (15) for freezing on tissue (3),
- a probe shank (13) for guiding the probe head (15) onto the tissue (3),
- a feed device (20) for feeding a fluid into an expansion chamber (50) of the instrument,
- a discharge device (40), which is connected to the expansion chamber (50) for discharging the fluid, wherein the expansion chamber (50) is designed in such a way that the supplied fluid expands therein for cooling the probe head (15),
wherein the discharge device (40) comprises at least one opening (41 - 41"') in the distal region of the probe (10) which discharges the fluid into an outer region outside the probe (10), and the cryosurgical instrument comprises an adapter for draining the fluid in the proximal direction, **characterised in that** the adapter comprises a protective sleeve (90) with a seal (91), wherein the probe (10) is movably supported within the protective sleeve (90) and the seal (91) distally seals an internal space of the protective sleeve (90), for receiving the fluid, in relation to the probe (10).

2. Cryosurgical instrument according to Claim 1,
**characterised in that**
the discharge device (40) comprises at least one opening (41 - 41"') at the distal end of the probe shank (13) and/or in the probe head (15).

3. Cryosurgical instrument according to one of the preceding claims,
**characterised in that**
the expansion chamber (50) for cooling the probe head (15) is located inside the probe (10).

4. Cryosurgical instrument according to one of the preceding claims,
**characterised in that**
the protective sleeve (90) comprises a receiving section (93) for the tissue sample (5).

5. Cryosurgical instrument according to one of the preceding claims,
**characterised in that**
the adapter is adapted for introducing the fluid into a working channel of an endoscope (80).

6. Cryosurgical instrument according to one of the preceding claims,
**characterised in that**
the feed device (20) comprises a feed line (21) which is arranged inside the probe shank (13).

7. Cryosurgical instrument according to one of the preceding claims,
**characterised in that**
the discharge device (40) comprises a particle filter on the probe head (15).

## Revendications

1. Instrument cryochirurgical pour le prélèvement d'un échantillon de tissu (5) avec une sonde (10), ladite sonde (10) comprenant :
- une tête (15) pour appliquer un froid extrême sur le tissu (3) ;
- une tige (13) pour amener la tête (15) de la sonde au niveau du tissu (3) ;
- un dispositif d'amenée (20) pour amener un fluide dans une chambre d'expansion (50) de l'instrument ;
- un dispositif d'évacuation (40) raccordé à la chambre d'expansion (50) en vue de l'évacuation du fluide, la chambre d'expansion (50) étant réalisée de telle sorte que le fluide amené est expansé dans celle-ci sous l'effet du refroidissement de la tête (15) de la sonde,
ledit dispositif d'évacuation (40) comprenant dans la zone distale de la sonde (10) au moins un orifice (41 - 41"'), par lequel le fluide est évacué vers une zone extérieure à l'extérieur de la sonde (10), et l'instrument cryochirurgical comprenant un adaptateur pour dévier le fluide dans la direction proximale,
**caractérisé en ce que**
l'adaptateur comprend un tuyau flexible de protection (90) avec un joint d'étanchéité (91), la sonde (10) étant logée de manière mobile dans le tuyau flexible de protection (90), et un espace intérieur du tuyau flexible de protection (90), destiné à recevoir le fluide, étant rendu étanche en distal par rapport à la sonde (10) par le joint d'étanchéité (91).

2. Instrument cryochirurgical selon la revendication 1, **caractérisé en ce que** le dispositif d'évacuation (40) comprend au moins un orifice (41 - 41"') au niveau de l'extrémité distale de la tige (13) de la sonde et/ou dans la tête (15) de la sonde.

3. Instrument cryochirurgical selon l'une quelconque des revendications précédentes, **caractérisé en ce que** la chambre d'expansion (50) destinée à refroidir la tête (15) de la sonde est disposée à l'intérieur de la sonde (10).

4. Instrument cryochirurgical selon l'une quelconque des revendications précédentes, **caractérisé en ce que** le tuyau flexible de protection (90) comprend une zone de réception (93) pour l'échantillon de tissu (5).

5. Instrument cryochirurgical selon l'une quelconque des revendications précédentes, **caractérisé en ce que** l'adaptateur est ajusté pour introduire le fluide dans un conduit de travail d'un endoscope (80).

6. Instrument cryochirurgical selon l'une quelconque des revendications précédentes, **caractérisé en ce que** le dispositif d'amenée (20) comprend une conduite d'amenée (21) disposée à l'intérieur de la tige (13) de la sonde.

7. Instrument cryochirurgical selon l'une quelconque des revendications précédentes, **caractérisé en ce que** le dispositif d'évacuation (40) comprend un filtre à particules au niveau de la tête (15) de la sonde.
